# EUROPEAN PATENT SPECIFICATION

(11) **EP 3 632 891 B1**
(45) Date of publication and mention of the grant of the patent: **17.07.2024**
(21) Application number: 18806145.1
(22) Date of filing: 04.05.2018
(51) Int. Cl.: C07C 69/67, C07C 279/14, A61K 8/37, A61K 31/21, A61Q 19/00, C07C 67/62

(54) **COMPOSITION CONTAINING ALKYL LACTATE**
ZUSAMMENSETZUNG MIT ALKYLLACTAT
COMPOSITION CONTENANT DU LACTATE D'ALKYLE

(30) Priority: 22.05.2017 KR 20170062881
(43) Date of publication of application: 08.04.2020
(73) Proprietor: GS Caltex Corporation, Seoul 06141 (KR)
(72) Inventor: LEE, Kyoung-Jun, Daejeon 34021 (KR); KIM, Woo-Young, Cheongju-si Chungcheongbuk-do 28427 (KR); CHOI, In-Chang, Daejeon 34070 (KR)
(74) Representative: Zacco Sweden AB
(86) International application number: PCT/KR2018/005224
(87) International publication number: WO 2018/216926

(56) References cited:
- EP-A1- 3 023 090
- DE-A1- 102010 030 555
- DE-A1- 2 746 108
- JP-A- 2002 020 231
- JP-A- 2003 519 111
- KR-A- 20160 017 198
- US-A- 4 540 567
- US-A1- 2009 253 668
- US-B2- 6 998 371

## Description

### Field

The present disclosure relates to an environmentally friendly ethyl lactate composition containing a basic amino acid as an anti-hydrolysis agent to inhibit hydrolysis of the ethyl lactate.

### Description of Related Art

Ethyl lactate is a representative environmentally friendly solvent that is non-toxic and biodegradable and is well mixed with water. Ethyl lactate may be applied to various applications such as semiconductor cleaners, cosmetics, and agriculture. Substances used in cosmetics, agriculture, etc. are required to be non-toxic because they can affect a human body. Accordingly, ethyl lactate is mainly used as a solvent in cosmetics, agriculture, and the like.

In this connection, when ethyl lactate is present with water, a content of ethyl lactate is reduced due to a hydrolysis reaction of ethyl lactate. Thus, it is difficult to preserve and use a constant amount of ethyl lactate.

EP3023090 A1 discloses a composition comprising alkyl lactate, water and amino acid.

DE2746108 A1 discloses a cosmetically acceptable ethyl lactate-containing composition for topical application to human skin.

US2009/253668 A1 shows an acne treatment composition comprising ethyl lactate.

US4540567 A shows a cosmetically acceptable composition containing a C1-C4 alkyl lactate dissolved in a mixture of water and a water-miscible C2-C4 alkylene glycol or a polymer thereof.

### DISCLOSURE

### TECHNICAL PURPOSES

One implementation of the present disclosure provides an environmentally friendly alkyl lactate composition that inhibits hydrolysis of alkyl lactate.

### TECHNICAL SOLUTIONS

In one implementation of the present disclosure, there is provided a composition containing an alkyl lactate, an anti-hydrolysis agent and water, wherein the anti-hydrolysis agent includes a basic amino acid.

### TECHNICAL EFFECTS

The composition contains a basic amino acid as an anti-hydrolysis agent to effectively inhibit the hydrolysis of alkyl lactate, and is harmless to the human body and thus may be used for cosmetics, agriculture, and the like.

### BRIEF DESCRIPTION OF DRAWINGS

FIG. 1 is a graph showing a degree of hydrolysis of ethyl lactate based on a water content.
FIG. 2 is a graph showing a degree of hydrolysis of each of compositions of Comparative Example 1 and Comparative Example 2.
FIG. 3 is a graph showing a hydrolysis degree of each of compositions of Comparative Example 1 and Present Examples 1 to 3 measured at a weekly interval.

### DETAILED DESCRIPTIONS

Benefits and features of the present disclosure, and how to achieve them will be apparent with reference to embodiments below. However, the present disclosure is not limited to embodiments as disclosed below, but may be implemented in various forms. Only embodiments are provided to ensure that the present disclosure is complete, and to fully inform a scope of the present disclosure to those of ordinary skill in the art. The scope of the present disclosure is defined only by claims.

The composition of the present disclosure contains ethyl lactate, an anti-hydrolysis agent and water, wherein the anti-hydrolysis agent is a basic amino acid selected from a group consisting of arginine, lysine, histidine, and combinations thereof, wherein a pH of the composition at room temperature is in a range of 6.5 to 7.5, and wherein a content of the anti-hydrolysis agent is in a range of 0.1 wt% to 5 wt%.

The composition contains a basic amino acid as an anti-hydrolysis agent to effectively inhibit the hydrolysis of ethyl lactate, and is harmless to the human body and thus may be used for cosmetics, agriculture, and the like.

. The ethyl lactate may be used in various fields as a representative green solvent that is nontoxic and biodegradable and well mixed with water.

The ethyl lactate may be a product of an esterification reaction of lactic acid and alcohol. For example, the ethyl lactate may be produced by esterification of lactic acid and ethanol as shown in a reaction scheme 1 below:

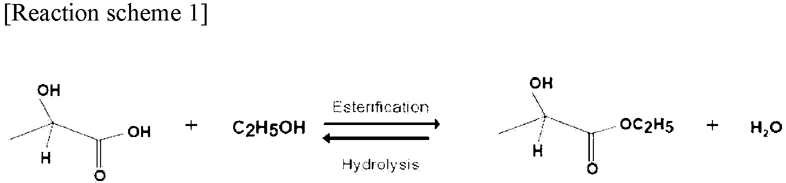

In this connection, when the ethyl lactate produced as described above is present with water, a hydrolysis reaction as a reverse reaction of the esterification may proceed spontaneously such that the product decomposes back into lactic acid and alcohol. Accordingly, a content of ethyl lactate in an initial storage and an ethyl lactate content after a certain period of time may be different from each other. Thus, there is a problem that it is difficult to store and use a constant amount of ethyl lactate.

The composition contains the basic amino acid as the anti-hydrolysis agent, together with the ethyl lactate and water. Accordingly, even though the ethyl lactate is present with water, the hydrolysis reaction can be effectively suppressed to maintain the ethyl lactate content above a certain level. In addition, since the basic amino acid is non-toxic and biodegradable, the composition containing the same may be used as a solvent for various applications.

Specifically, the basic amino acid may include one selected from a group consisting of arginine, lysine, histidine, and combinations thereof. The amino acid may include an amino acid of a type D or L.

The composition contains about 0.1% to about 5% by weight of the anti-hydrolysis agent, and specifically, about 0.1% to about 3% by weight of the anti-hydrolysis agent. The composition contains the anti-hydrolysis agent in the content of the above range, thereby to effectively inhibit the hydrolysis reaction of the ethyl lactate contained in the composition and to maintain the content of the ethyl lactate at a certain level or greater.

Specifically, when the content of the anti-hydrolysis agent is smaller than the above range, the anti-hydrolysis agent does not effectively inhibit the hydrolysis reaction of the ehtyl lactate, thereby reducing the content of ethyl lactate, and thus the lactic acid produced by the hydrolysis reaction may cause the hydrolysis reaction to be accelerated in series. To the contrary, when the content of the anti-hydrolysis agent exceeds the above range, the composition turns into a basic state to change physical properties of the composition containing the anti-hydrolysis agent such that an unwanted reaction occur when using the composition.

The composition may contain about 0.1% to about 89.9% by weight of water, and may contain about 10% to about 99.8% by weight of the ethyl lactate. The composition contains ethyl lactate and water in the above ranges, thereby effectively inhibiting the hydrolysis reaction of the ethyl lactate contained in the composition such that the ethyl lactate may be maintained and used at a constant concentration and a constant content.

The composition has a pH of about 6.5 to about 7.5 at room temperature. The composition is maintained at the pH of the above range, thereby effectively inhibiting the hydrolysis reaction of the ethyl lactate contained in the composition to maintain the content of the ethyl lactate above a certain level. Specifically, when the pH of the composition is out of the above range, there are many protons or anions that may cause a side reaction of the ethyl lactate such that physical properties of the composition changes, and the hydrolysis reaction proceeds to cause generation of the lactic acid.

For the composition above, a hydrolysis percentage of the ethyl lactate in the composition containing 50% by weight of water may be defined based on contents of ethyl lactate before and after preservation of the composition for 4 to 6 weeks at 20 °C to 40 °C and is calculated based on a following equation 1, and is lower than 2 %: Hydrolysis percentage (%) = [(content of ehtyl lactate before preservation (g) - content of ethyl lactate after preservation (g)) / content of ethyl lactate before preservation (g)] × 100

The composition has a low hydrolysis percentage of the ethyl lactate in the above range. Thus, it is easy to preserve the ethyl lactate in a constant concentration and content therein. Accordingly, the composition containing the ethyl lactate may be easily used.

The composition may be applied as a cosmetic composition; an agricultural composition; a pharmaceutical compositions; or an industrial composition.

In general, when alkyl lactate, specifically ethyl lactate, is used in cosmetics or agriculture, the ethyl lactate is mixed with water. As described above, when ethyl lactate is used together with water, the hydrolysis reaction as a reverse reaction of esterification spontaneously proceeds, such that the ethyl lactate is decomposed back into lactic acid and alcohol. Accordingly, since the content of ethyl lactate at a beginning time of use and the content of ethyl lactate after a certain period of time may be different from each other, it is difficult to store and use a constant amount of ethyl lactate.

However, the composition in accordance with the present disclosure may contain the basic amino acid as an anti-hydrolysis agent to effectively inhibit the hydrolysis of ethyl lactate.

In addition, the composition containing only a non-toxic and environmentally friendly substance may be used without limitation in various applications. The composition contains ethyl lactate, anti-hydrolysis agent and water. The ethyl lactate is a non-toxic and biodegradable environmentally friendly solvent. The anti-hydrolysis agent as the basic amino acid is also a non-toxic biodegradable substance, and harmless to the human body. Thus, the composition may be useful as a cosmetic composition, an agricultural composition and a pharmaceutical composition.

Hereinafter, Present Examples of the present disclosure will be described. However, the Present Examples as described below are merely to specifically illustrate or explain the present disclosure, and thus the present disclosure should not be limited thereto.

### <Present Example and Comparative Example>

### Present Example 1

A composition containing ethyl lactate, 50% by weight of water, and 0.33% by weight of arginine was prepared. The prepared composition was sealed to prevent exposure to moisture and air and then was sufficiently stirred. The composition was stored at a temperature of about 40 °C in a convection oven.

### Present Example 2

A composition was prepared in the same manner as in Present Example 1 except for using lysine instead of arginine.

### Present Example 3

A composition was prepared in the same manner as in Example 1 except that histidine was used instead of arginine.

### Comparative Example 1

A composition free of arginine was prepared to have ethyl lactate and 50% by weight of water.

### Comparative Example2

A composition was prepared in the same manner as in Present Example 1 except that sodium hydroxide was used instead of arginine.

### <Evaluation>

### Experimental Example 1: Hydrolysis Degree of Ethyl Lactate based on Water Content

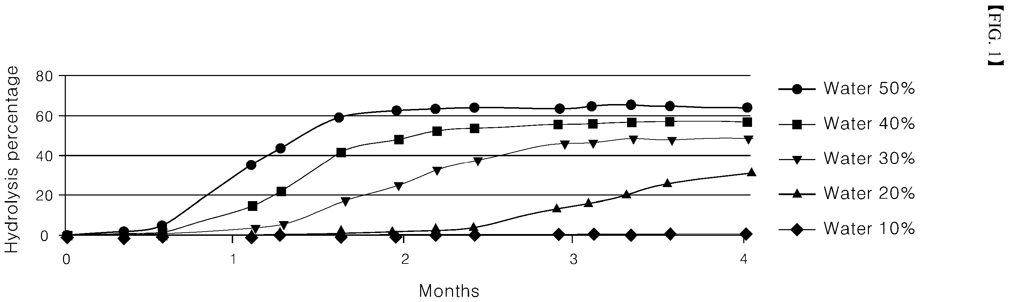

As the water content in the composition containing ethyl lactate and free of a separate anti-hydrolysis agent was increased from 10 wt% to 50 wt%, the degree of hydrolysis of ethyl lactate at room temperature was measured and shown in FIG. 1.

Specifically, the amounts of lactic acid and ethanol contained in the composition were measured every month to indicate the degree of hydrolysis of ethyl lactate.

The composition was measured by gas chromatography. GC 7890 from Agilent and Rtx-VRX from GC column Restek were used as measurement devices.

As shown in FIG. 1, the hydrolysis percentage increases rapidly as the water content in the composition increases.

### Experimental Example 2: Hydrolysis Degree of Ethyl Lactate based on Water Content

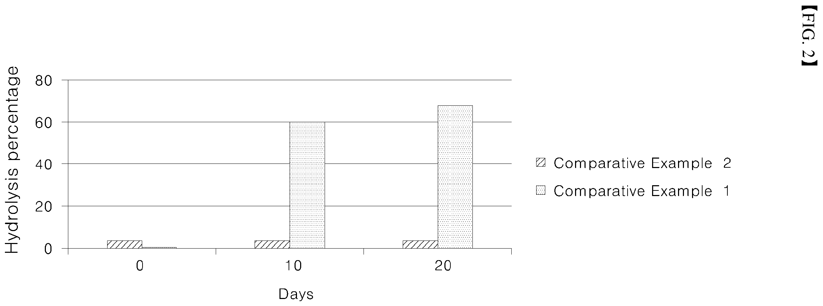

At 40 ° C, the degree of hydrolysis of each of the compositions of the Comparative Example 1 and Comparative Example 2 was measured and shown in FIG. 2.

The hydrolysis degree of ethyl lactate was measured by measuring the content of lactic acid and ethanol generated in the composition due to a hydrolysis reaction. The composition was measured by gas chromatography. GC 7890 from Agilent and Rtx-VRX from GC column Restek were used as measurement devices.

### Experimental Example 3: Hydrolysis Degree of Ethyl Lactate based on Water Content

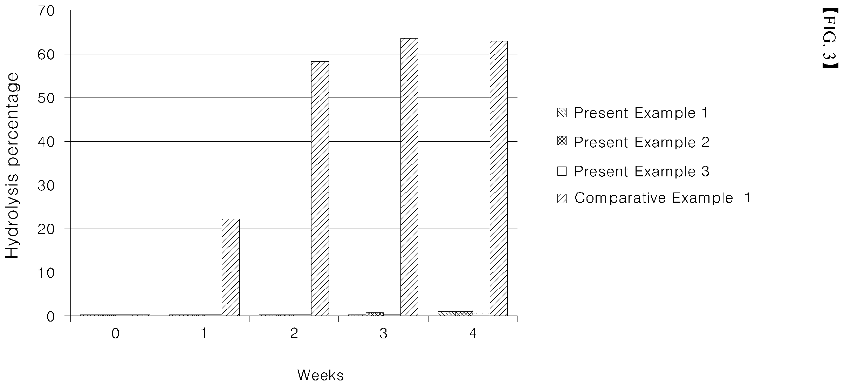

Hydrolysis of each of the compositions of Comparative Example 1 and Present Examples 1 to 3 at 40 °C was measured for 4 weeks at 1 week interval and is shown in FIG. 3. The hydrolysis degree of ethyl lactate was measured by measuring the content of lactic acid and ethanol generated in the composition due to a hydrolysis reaction.

The hydrolysis percentages of the compositions of Comparative Example 1 and Present Examples 1 to 3 for 4 weeks are shown in Table 1 below. The hydrolysis percentages of the compositions were calculated based on the following equation 1. Each composition was measured by gas chromatography. GC 7890 from Agilent and Rtx-VRX from GC column Restek were used as measurement devices. Hydrolysis percentage (%) = [(content of alkyl lactate before preservation (g) - content of alkyl lactate after preservation (g)) / content of alkyl lactate before preservation (g)] × 100

**[Table 1]**

| | Hydrolysis percentage (%) |
|---|---|
| Present Example 1 | 1.0 |
| Present Example 2 | 1.0 |
| Present Example 3 | 1.3 |
| Comparative Example 1 | 62.9 |

In FIG. 2, the composition of Comparative Example 2 using sodium hydroxide as an anti-hydrolysis agent has a lower hydrolysis percentage compared to the composition of Comparative Example 1 without an anti-hydrolysis agent. After about 20 days, the composition of Comparative Example 2 shows a hydrolysis percentage of about 4%.

As shown in Table 1, the compositions of the Present Examples containing a basic amino acid as an anti-hydrolysis agent have a significantly low hydrolysis percentage of about 1.0% after 4 weeks.

That is, each of the compositions of the Present Examples contains a basic amino acid as an anti-hydrolysis agent which is non-toxic and biodegradable to effectively inhibit the hydrolysis of alkyl lactate, and is harmless to the human body and thus may be used for various applications.

## Claims

1. A composition containing ethyl lactate, an anti-hydrolysis agent, and water, wherein the anti-hydrolysis agent is a basic amino acid selected from a group consisting of arginine, lysine, histidine, and combinations thereof, and wherein a pH of the composition at room temperature is in a range of 6.5 to 7.5, wherein a content of the anti-hydrolysis agent is in a range of 0.1 wt% to 5 wt%.

2. The composition of claim 1, wherein a content of the water is in a range of 0.1 wt% to 89.9 wt%.

3. The composition of claim 1, wherein a content of the ethyl lactate is in a range of 10 wt% to 99.8 wt%.

4. The composition of claim 1, wherein the composition includes a cosmetic composition, an agricultural composition, a pharmaceutical composition, or an industrial composition.

5. The composition of claim 1, wherein a hydrolysis percentage of the ethyl lactate in the composition containing 50% by weight of water is defined based on contents of ethyl lactate before and after preservation of the composition for 4 to 6 weeks at 20 °C to 40 °C and is calculated based on a following equation 1, and is lower than 2 %: Hydrolysis percentage (%) = [(content of ethyl lactate before preservation (g) - content of ethyl lactate after preservation (g)) / content of ethyl lactate before preservation (g)] × 100

## Patentansprüche

1. Zusammensetzung, die Ethyllactat, ein hydrolysehemmendes Mittel und Wasser enthält, wobei das hydrolysehemmende Mittel eine basische Aminosäure ist, die aus einer Gruppe ausgewählt ist, die aus Arginin, Lysin, Histidin und Kombinationen davon besteht, und wobei ein pH-Wert der Zusammensetzung bei Raumtemperatur in einem Bereich von 6,5 bis 7,5 liegt, wobei ein Gehalt des hydrolysehemmenden Mittels in einem Bereich von 0,1 Gew.-% bis 5 Gew.-% liegt.

2. Zusammensetzung nach Anspruch 1, wobei ein Gehalt an dem Wasser in einem Bereich von 0,1 Gew.-% bis 89,9 Gew.-% liegt.

3. Zusammensetzung nach Anspruch 1, wobei ein Gehalt an dem Ethyllactat in einem Bereich von 10 Gew.-% bis 99,8 Gew.-% liegt.

4. Zusammensetzung nach Anspruch 1, wobei die Zusammensetzung eine kosmetische Zusammensetzung, eine landwirtschaftliche Zusammensetzung, eine pharmazeutische Zusammensetzung oder eine industrielle Zusammensetzung einschließt.

5. Zusammensetzung nach Anspruch 1, wobei ein Hydrolyseprozentsatz des Ethyllactats in der Zusammensetzung, die zu 50 Gew.-% Wasser enthält, auf der Grundlage des Gehalts an Ethyllactat vor und nach Konservierung der Zusammensetzung für 4 bis 6 Wochen bei 20 °C bis 40 °C definiert ist und auf der Grundlage einer nachstehenden Gleichung 1 berechnet ist und weniger als 2 % beträgt: Hydrolyseprozentsatz (%) = [Gehalt an Ethyllactat vor Konservierung (g) - Gehalt an Ethyllactat nach Konservierung (g)) / Gehalt an Ethyllactat vor Konservierung (g)] x 100

## Revendications

1. Composition contenant du lactate d'éthyle, un agent anti-hydrolyse et de l'eau, dans laquelle l'agent anti-hydrolyse est un acide aminé basique choisi dans un groupe constitué de l'arginine, de la lysine, de l'histidine et de leurs combinaisons, et dans laquelle un pH de la composition à température ambiante est compris dans une plage de 6,5 à 7,5, dans laquelle la teneur en agent anti-hydrolyse est comprise dans une plage de 0,1 % en poids à 5 % en poids.

2. Composition selon la revendication 1, dans laquelle la teneur en eau est comprise dans une plage de 0,1 % en poids à 89,9 % en poids.

3. Composition selon la revendication 1, dans laquelle la teneur en lactate d'éthyle est comprise dans une plage de 10 % en poids à 99,8 % en poids.

4. Composition selon la revendication 1, dans laquelle la composition comporte une composition cosmétique, une composition agricole, une composition pharmaceutique ou une composition industrielle.

5. Composition selon la revendication 1, dans laquelle un pourcentage d'hydrolyse du lactate d'éthyle dans la composition contenant 50 % en poids d'eau est défini sur la base de teneurs en lactate d'éthyle avant et après conservation de la composition pendant 4 à 6 semaines à une température de 20 °C à 40 °C et est calculé sur la base de l'équation 1 suivante, et est inférieur à 2 % : pourcentage d'hydrolyse (%) = [(teneur en lactate d'éthyle avant conservation (g) = teneur en lactate d'éthyle après conservation (g)) / teneur en lactate d'éthyle avant conservation (g)] × 100
